# EUROPÄISCHE PATENTANMELDUNG

(11) **EP 4 763 241 A1**
(43) Veröffentlichungstag der Anmeldung: **24.06.2026**
(21) Anmeldenummer: 25224319.1
(22) Anmeldetag: 17.12.2025
(51) Int. Cl.: A61M 1/14, A61M 39/10

(54) **EXTRAKORPORALE BLUTBEHANDLUNGSMASCHINE UND FLUIDISCHES VERBINDUNGSSTÜCK DAFÜR**

(30) Priorität: 20.12.2024 DE 102024139360
(71) Anmelder: B. Braun Avitum AG, 34212 Melsungen (DE)
(72) Erfinder: KNIERIM, Michael, 37242 Bad Sooden-Allendorf (DE); MEIERHOLZ, Katharine, 34212 Melsungen (DE)
(74) Vertreter: Winter, Brandl - Partnerschaft mbB

(57) **Zusammenfassung**

Die Offenbarung betrifft eine extrakorporale Blutbehandlungsmaschine (1), insbesondere Dialysemaschine, mit Fluidleitungen (8, 10), die jeweils in Abschnitte (8b, 8c, 10b, 10c) segmentiert vorgesehen und ausgestaltet sind, und elektrisch leitfähigen Fluidkanälen (32, 34), über die jeweils zwei Abschnitte (8b, 8c, 10b, 10c) der jeweiligen Fluidleitung (8, 10) fluidisch verbunden sind, wobei die Fluidkanäle (32, 34) zur elektrischen Verbindung mit einem elektrischen Bezugspotential (16) vorgesehen sind oder mit einem elektrischen Bezugspotential (16) elektrisch verbunden sind, dadurch gekennzeichnet, dass wenigstens zwei der Fluidkanäle (32, 34) zu einem elektrisch leitfähigen, fluidischen Verbindungsstück (14; 114; 214) zusammengefasst sind. Weiterhin betrifft die Offenbarung ein elektrisch leitfähiges, fluidisches Verbindungsstück für eine oder einer extrakorporalen Blutbehandlungsmaschine.

## Beschreibung

### Technisches Gebiet

Die vorliegende Offenbarung betrifft eine extrakorporale Blutbehandlungsmaschine, insbesondere eine Dialysemaschine, zur Hämodialyse, Hämofiltration, Hämodiafiltration und/oder Ultrafiltration, sowie ein fluidisches Verbindungsstück für eine extrakorporale Blutbehandlungsmaschine.

Eine extrakorporale Blutbehandlungsmaschine hat einen Dialysator mit einer semipermeablen Membran für einen Stoffaustausch zwischen dem in einem extrakorporalen Blutkreislauf geförderten Blut eines Patienten und einer in einem Dialysierflüssigkeitskreislauf geförderten Dialysierflüssigkeit. Darüber hinaus sind für einen bestimmungsgemäßen Betrieb weitere, fluidführende Kreisläufe vorgesehen, wie beispielsweise ein Substituat-Kreislauf oder ein zentraler (CCS) oder dezentraler Konzentrat-Kreislauf.

### Technischer Hintergrund

Bei einer extrakorporalen Blutbehandlung, beispielsweise einer Blutreinigung in Form einer Hämodialyse, Hämofiltration oder Hämodiafiltration, wird einem Dialysepatienten Blut über einen arteriellen Gefäßzugang entnommen und über einen extrakorporalen Blutkreislauf einem Dialysator für eine Blutbehandlung zugeführt. Dem Dialysator wird zudem eine Dialysierflüssigkeit über einen separaten Dialysierflüssigkeitskreislauf zugeführt. In dem Dialysator werden das Blut des Blutkreislaufs und die Dialysierflüssigkeit des Dialysierflüssigkeitskreislaufs über eine semipermeable Membran in Kontakt gebracht, so dass ein Stoffaustausch zwischen dem Blut und der Dialysierflüssigkeit stattfinden kann. So können bei der Dialysebehandlung niereninsuffizienter Patienten Schadstoffe aus dem Blut sowie auch überschüssiges Wasser, welches sich beispielsweise aufgrund eines zugrundeliegenden Nierenversagens im Körper ansammelt, entfernt werden. Das so gereinigte Blut wird anschließend über einen venösen Gefäßzugang an den Patienten wieder zurückgeführt.

Fluidführende Kreisläufe, wie beispielsweise derjenige der Dialysierflüssigkeit, des Substituats oder des Konzentrats, bergen das Risiko einer hydrostatischen Aufladung. Neben dieser Aufladung stellt insbesondere ein elektrischer Fehlerfall ein Risiko dar, das vermieden werden muss. Um dem elektrischen Fehlerfall innerhalb der Blutbehandlungsmaschine vorzubeugen und insbesondere, um einen Patientenableitstrom einzuhalten/ zu begrenzen, sind die fluidführenden Leitungen oder Bauteile erdungspflichtig, insbesondere, wenn sie in Richtung des Patienten führen.

Zur Erdung fluidführender Leitungen eines Dialysegerätes schlägt das Patent EP 1 633 428 B1 vor, elektrisch leitfähige, fluidische Verbindungsstücke zu nutzen und mit Masse zu verbinden. An das fluidische Verbindungsstück sind jeweils dielektrische Abschnitte der fluidführenden Leitung angebunden, sodass das Verbindungsstück einen Abschnitt des fluidischen Strömungspfades der Leitung ausbildet. Das Verbindungsstück ist in ein Stützelement eingeclipst, dessen elektrisch leitfähiger Abschnitt mit Masse verbunden ist. Das Stützelement ist wiederum an einem Gehäuseabschnitt befestigt.

Nachteilig daran ist, dass diese Art der Erdung und Befestigung fluidführender Leitungen einen erheblichen vorrichtungstechnischen Aufwand darstellt.

### Kurzbeschreibung der vorliegenden Offenbarung

Die Aufgabe der vorliegenden Offenbarung ist demgegenüber, die Nachteile aus dem Stand der Technik zu vermeiden oder zumindest zu mindern und eine extrakorporale Blutbehandlungsmaschine bereitzustellen, bei der eine Erdung erdungspflichtiger, fluidführender Leitungen mit geringerem vorrichtungstechnischen Aufwand erfolgt. Darüber hinaus besteht die Aufgabe darin, ein elektrisch leitfähiges Verbindungsstück bereitzustellen, über das eine Erdung erdungspflichtiger, fluidführender Leitungen einer extrakorporalen Blutbehandlungsmaschine mit geringerem vorrichtungstechnischen Aufwand erfolgen kann.

Die Aufgabe wird offenbarungsgemäß durch eine extrakorporale Blutbehandlungsmaschine mit den Merkmalen des Anspruchs 1, sowie durch ein elektrisch leitfähiges Verbindungsstück mit den Merkmalen des Anspruchs 10 gelöst.

Weiterbildungen der extrakorporalen Blutbehandlungsmaschine, sowie des elektrisch leitfähigen Verbindungsstücks sind in den abhängigen Ansprüchen beansprucht und/oder nachfolgend beschrieben.

Ein Grundgedanke der vorliegenden Offenbarung sieht vor, dass die Erdung von mehreren, insbesondere erdungspflichtigen, Fluidleitungen einer extrakorporalen Blutbehandlungsmaschine an einem elektrisch leitfähigen, fluidführenden Verbindungsstück in baulicher Einheit zusammengefasst ist. Hierzu hat das Verbindungsstück für mehrere Fluidleitungen je einen elektrisch leitfähigen Fluidkanal. Die Fluidkanäle bilden eine bauliche Einheit, wobei an die Fluidkanäle Abschnitte der erdungspflichtigen Fluidleitungen fluidisch anbindbar oder angebunden sind.

Konkret weist eine extrakorporale Blutbehandlungsmaschine gemäß der vorliegenden Offenbarung, insbesondere eine Dialysemaschine, insbesondere Hämodialysemaschine, zumindest auf:
- Fluidleitungen, die jeweils in Abschnitte segmentiert sind, und bevorzugt während eines bestimmungsgemäßen Einsatzes der Blutbehandlungsmaschine, also im Rahmen der extrakorporalen Blutbehandlung, erdungspflichtig sind, und
- elektrisch leitfähige Fluidkanäle, über die jeweils zwei Abschnitte der jeweiligen Fluidleitung fluidisch verbunden sind, wobei die Fluidkanäle zur elektrischen Verbindung mit einem elektrischen Bezugspotential vorgesehen, vorzugsweise vorbereitet, sind oder mit dem elektrischen Bezugspotential elektrisch verbunden sind.

Erfindungsgemäß sind wenigstens zwei der Fluidkanäle zu einem elektrisch leitfähigen, fluidischen Verbindungsstück zusammengefasst, insbesondere in baulicher Einheit zusammengefasst.

Auf diese Weise können gleich mehrere Fluidleitungen über das eine fluidische Verbindungsstück mit dem elektrischen Bezugspotential verbunden werden, insbesondere geerdet werden, indem sie gemeinsam an dasselbe Verbindungsstück fluidisch angebunden werden. Eine Anzahl von an das Bezugspotential anzuschließenden Verbindungsstücken kann so minimiert werden, was den vorrichtungstechnischen Aufwand zur Erdung und zur Befestigung von Fluidleitungen minimiert, was wiederum eine Kostenreduktion darstellt.

In anderen Worten sind in das eine fluidische Verbindungsstück - bzw., in das eine elektrisch leitfähige, fluidische Bauteil - mehrere zu erdende oder erdungspflichtige Fluidleitungen fluidisch integriert.

Besonders vorteilhaft ist es, wenn die Abschnitte zu erdender oder erdungspflichtiger Fluidleitungen, die ohnehin paarweise, bündelweise oder in unmittelbarer Nähe zueinander verlaufen, an dasselbe Verbindungsstück fluidisch angebunden sind.

Das fluidische Verbindungsstück weist in Summe vorzugsweise zwei, drei, vier oder mehr Fluidkanäle auf. Eine Anzahl kann gerade oder ungerade sein.

Gemäß einer Weiterbildung hat die Blutbehandlungsmaschine ein Gehäuse, in welchem die erdungspflichtigen Fluidleitungen zumindest abschnittsweise verlaufen, insbesondere wenigstens eine der Fluidleitungen eines Dialysierflüssigkeitskreislaufs der extrakorporalen Blutbehandlungsmaschine.

Gemäß einer Weiterbildung ist das Verbindungsstück zumindest abschnittsweise, insbesondere vollständig, in einem von dem Gehäuse der extrakorporalen Blutbehandlungsmaschine umfangenen/ umfassten/ umgebenen Innenraum angeordnet.

Bei der genannten vollständigen Anordnung im Innenraum erfolgt der Anschluss der erdungspflichtigen Fluidleitungen an das Bezugspotential (Erdung, insbesondere funktionale Erdung) über das Verbindungsstück vollständig innerhalb des Gehäuses, das heißt ohne Kontakt zum Gehäuse und somit ohne Patientenrisiko. Bei der vollständigen Anordnung des fluidischen Verbindungsstücks im Innenraum ist das Verbindungsstück weder an der Gehäusewandung angeordnet, noch durchsetzt oder durchdringt es die Gehäusewandung. Eine elektrische Isolierung des fluidischen Verbindungsstücks gegen das Gehäuse kann entfallen.

Eine Befestigung des fluidischen Verbindungsstücks kann im vom Gehäuse umfangenen Innenraum ggf. mit geringerem vorrichtungstechnischem Aufwand erfolgen, als im Falle der Befestigung in oder an einer Gehäuseausnehmung.

Alternativ ist das Verbindungsstück an dem Gehäuse/ an der Gehäusewandung angebracht/ befestigt.

Dazu kann das Verbindungsstück in einer Ausnehmung, insbesondere Durchgangsausnehmung oder Sacklochausnehmung, des Gehäuses oder der Gehäusewandung angeordnet oder befestigt sein. Beispielsweise kann das Verbindungsstück die Durchgangsausnehmung mit Endabschnitten der Fluidkanäle durchsetzen. Alternativ oder ergänzend ist das Verbindungsstück mittels einer Ausnehmung, insbesondere Durchgangsausnehmung oder Sacklochausnehmung oder Sicke, des Gehäuses oder der Gehäusewandung angeordnet oder befestigt.

Das Verbindungsstück ist offenbarungsgemäß flexibel anordenbar oder angeordnet, wie oben dargelegt beispielsweise: in dem Gehäuse, an einer Komponente innerhalb des Gehäuses, an dem Gehäuse, vollständig in dem Innenraum des Gehäuses, an einer zumindest abschnittsweise innerhalb des Gehäuses angeordneten Komponente oder die Gehäusewandung durchdringend. Das hat den Vorteil, dass ein Erdungsanschluss der extrakorporalen Blutbehandlungsmaschine flexibel positionierbar ist, was Vorteile in der Bauraumausnutzung und der Ausgestaltung der Blutbehandlungsmaschine bringt.

Insbesondere kann das Verbindungsstück zur Erdung bei leitfähigen Gehäusen und auch bei nicht leitfähigen Gehäusen genutzt werden.

Gemäß einer Weiterbildung sind mehrere offenbarungsgemäße Verbindungsstücke vorgesehen, und insbesondere verteilt angeordnet.

Das jeweilige Verbindungsstück ist vorzugsweise bedarfsgerecht angeordnet, das heißt abschnittsweise oder vollständig im Innenraum oder eine Gehäusewandung durchsetzend.

Die extrakorporale Blutbehandlungsmaschine weist insbesondere auf:
- einen Dialysierflüssigkeitskreislauf, der dafür angepasst ist, ein Fluid, insbesondere Dialysierflüssigkeit, entlang einer Dialysierflüssigkeitsseite zu fördern und insbesondere verbrauchte Dialysierflüssigkeit bzw. Dialysat abzuführen.

Weiter bevorzugt kann die extrakorporale Blutbehandlungsmaschine ferner aufweisen:
- einen Dialysator mit einer semipermeablen Membran mit einer Blutseite und der Dialysierflüssigkeitsseite, und
- einen extrakorporalen Blutkreislauf, der dafür angepasst ist, ein Fluid, insbesondere Blut, entlang der Blutseite zu fördern.

Der extrakorporale Blutkreislauf erstreckt sich insbesondere von einem arteriellen Abschnitt, welcher zur Konnektierung eines arteriellen Shunt-Abschnitts vorgesehen ist, über einen Bluteingang des Dialysators, durch den Dialysator, über einen Blutausgang des Dialysators zu einem venösen Abschnitt, welcher zur Konnektierung eines venösen Shunt-Abschnitts vorgesehen ist.

Der Dialysierflüssigkeitskreislauf erstreckt sich insbesondere von einer Dialysierflüssigkeitsbereitstellung der Blutbehandlungsmaschine zu einem Dialysierflüssigkeitseingang des Dialysators, zu einem Dialysatausgang des Dialysators und hin zu einer Dialysatsenke der Blutbehandlungsmaschine.

Vorzugsweise sind die Fluidkanäle des fluidischen Verbindungsstücks parallel zueinander angeordnet oder verlaufen parallel. Alternativ oder ergänzend sind die Fluidkanäle des fluidischen Verbindungsstücks zueinander angestellt oder gekreuzt angeordnet oder verlaufen zueinander angestellt oder gekreuzt.

Beispiele von zu erdenden oder erdungspflichtigen Fluidleitungen sind unter anderem:
- eine als Zulauf ausgebildete Fluidleitung und eine als Rücklauf ausgebildete Fluidleitung des Dialysierflüssigkeitskreislaufs, die vorzugsweise in einem Bereich einer Spülbrücke und/ oder Masseplatte der Blutbehandlungsmaschine angebracht sind;
- Fluidleitungen eines Substituatports der Blutbehandlungsmaschine; oder
- Fluidleitungen einer zentralen Konzentratversorgung (CCS) der Blutbehandlungsmaschine.

Gemäß einer Weiterbildung ist eine der Fluidleitungen ein Zulauf, über den die frische Dialysierflüssigkeit oder ein Vorprodukt frischer Dialysierflüssigkeit, insbesondere eine alkalisierende Lösung, bereitgestellt wird, und die andere Fluidleitung ist ein Ablauf, über den verbrauchte Dialysierflüssigkeit abgeführt wird.

Gemäß einer Weiterbildung ist eine der Fluidleitungen eine Substituatleitung, die vorgesehen und ausgestaltet ist, dem extrakorporalen Blutkreislauf - und damit dem Patienten - Substituat zuzuführen.

Es können mehrere Zuläufe und/ oder mehrere Abläufe als Fluidleitungen an dasselbe Verbindungsstück fluidisch angebunden sein.

Es können alle Fluidkanäle des Verbindungsstücks mit einer jeweiligen Fluidleitung fluidisch verbunden sein, sie müssen es aber nicht sein.

Gemäß einer Weiterbildung ist wenigstens einer der Fluidkanäle frei und/ oder zur fluidischen Verbindung mit einer Fluidleitung vorgehalten. Das ermöglicht die Verwendung eines, bezogen auf die Anzahl Fluidkanäle überdimensionierten, fluidischen Standard-Verbindungsstücks mit beispielsweise drei, vier, fünf oder sechs (oder mehr) Fluidkanälen. Zwar beansprucht ein derartiges Verbindungsstück im Falle einer Unterbelegung der Fluidkanäle - wenn beispielsweise nur vier von sechs möglichen Fluidleitungen angebunden sind - mehr Bauraum als ein spezifisch dimensioniertes Verbindungsstück. Jedoch kann die standardisierte Herstellung und Bereitstellung diesen Nachteil aufwiegen.

Gemäß einer bevorzugten Weiterbildung ist das fluidische Verbindungsstück einstückig, insbesondere einstückig gefertigt. Das heißt, dass es bevorzugt ohne Fügen gefertigt ist.

Gefertigt ist das fluidische Verbindungsstück insbesondere aus leitfähigem Kunststoff, insbesondere durch Spritzgießen, oder vorzugsweise aus Metall, insbesondere durch ein Metal Injection Moulding Verfahren (MIM).

Bei gezielter Auswahl des Metalls oder Kunststoffs sind die hohen Anforderungen an die Beständigkeit und Biokompatibilität des Materials, die im medizinischen Bereich gestellt werden, unter allen Bedingungen sicher erfüllt.

Die Fertigung per MIM Verfahren weist vorzugsweise folgende Schritte auf:
- Mischen eines Metallpulvers mit einem thermoplastischen Kunststoffbindemittel,
- Spritzgießen dieses Gemischs zu einem Rohling,
- Lösen und Entfernen des Kunststoffbindemittels vom Rohling,
- Sintern des Rohlings, und
- Bereitstellen des so gesinterten Verbindungsstücks.

Neben den geringen Fertigungskosten derartiger Urformverfahren ist ein weiterer Vorteil, dass mit lediglich einem Fertigungsschritt gleich mehrere Funktionen in das Verbindungsstück integriert sind. So können offenbarungsgemäß im gleichen Fertigungsschritt der Fluidkanal, dessen spezifische Anschlussgeometrie zur fluidischen Anbindung der Fluidleitung, ein Befestigungsabschnitt des Verbindungsstücks zur Befestigung innerhalb des Innenraums und ein Kontaktelement zur elektrischen Anbindung einer Masseleitung an das Verbindungsstück usw. ausgebildet werden.

Alternativ oder ergänzend kann ein spanendes Fertigungsverfahren zur Fertigung des fluidischen Verbindungsstücks angewendet werden.

Gemäß einer Weiterbildung hat das elektrisch leitfähige, fluidische Verbindungsstück einen Befestigungsabschnitt, über den es an einer im Innenraum angeordneten, mit dem elektrischen Bezugspotential verbundenen Komponente oder an einem Gehäuse der Blutbehandlungsmaschine befestigt ist.

Die Komponente kann vollständig in dem Innenraum angeordnet sein. Ein Beispiel einer solchen Komponente ist insbesondere eine im Innenraum angeordnete Masseplatte.

Alternativ kann die Komponente lediglich abschnittsweise in dem Innenraum angeordnet sein. Als Beispiel für eine derartige Komponente kann ein Substituatport der extrakorporalen Blutbehandlungsmaschine genannt werden, der einerseits einen nach innen weisenden Abschnitt und andererseits einen nach außen weisenden, von außen zugänglichen Abschnitt aufweist.

Im Falle des Substituatports ist die erdungspflichtige, an das Verbindungsstück angebundene Fluidleitung vorzugsweise eine Substituatleitung, die mit dem extrakorporalen Blutkreislauf verbunden ist.

Auf diese Weise erfolgen über den Befestigungsabschnitt sowohl die Befestigung des fluidischen Verbindungsstücks in dem Innenraum, als auch die elektrische Verbindung der wenigstens einen zu erdenden oder erdungspflichtigen Fluidleitung mit dem elektrischen Bezugspotential.

Gemäß einer Weiterbildung erstreckt sich der Befestigungsabschnitt zumindest abschnittsweise entlang dem wenigstens einen Fluidkanal, insbesondere radial zu dessen Erstreckungsrichtung, sodass der Befestigungsabschnitt zum Zwecke der Montage oder der Befestigung des fluidischen Verbindungsstücks - oder für eine entsprechende Demontage oder ein Lösen - leicht zugänglich ist.

Im Falle mehrerer Fluidkanäle erstreckt sich der Befestigungsabschnitt vorzugsweise zwischen zwei Fluidkanälen und bildet so einen Verbindungsabschnitt aus, über den die beiden Fluidkanäle mechanisch fest verbunden sind.

Gemäß einer möglichen Ausgestaltung ist die Komponente ein Blech, ein Rahmenteil, ein Trägerteil oder dergleichen, das in dem vom Gehäuse umfangenen Innenraum der Blutbehandlungsmaschine angeordnet ist.

Gemäß einer Weiterbildung hat der Befestigungsabschnitt eine, insbesondere zentrale, Durchgangsausnehmung, insbesondere Durchgangsbohrung, wobei die Durchgangsausnehmung vorzugsweise von einer Schraube durchsetzt ist, die in eine Gewindebohrung der Komponente eingeschraubt ist.

Zur Sicherung der Schraube kann vorzugsweise eine Zahnscheibe vorgesehen sein und/ oder die Schraube ist mit einer Verzahnung versehen.

Die Zahnscheibe oder Verzahnung kann abgesehen von der Sicherung Vorteile bezüglich der elektrischen Leitfähigkeit bieten.

Alternativ oder ergänzend zur Verschraubung kann der Befestigungsabschnitt einen, insbesondere zentralen, Vorsprung aufweisen, der in eine Ausnehmung oder Bohrung der Komponente eingesetzt, und insbesondere darin verklebt, ist. Selbstverständlich ist hierbei auch eine umgekehrte Verbindung möglich, das heißt, dass der Befestigungsabschnitt eine Ausnehmung hat, die einen Vorsprung der Komponente übergreift, und insbesondere mit diesem verklebt ist.

Um das fluidische Verbindungsstück auf einfache Weise gegen eine Verdrehung an der Komponente zu sichern, weist es, insbesondere an seinem Befestigungsabschnitt, ein zu der Durchgangsausnehmung oder zu dem Vorsprung beabstandetes, insbesondere dezentral oder exzentrisch angeordnetes, insbesondere als Ausnehmung oder Zapfen ausgebildetes, Positioniermittel auf, das in Eingriff mit einem an der Komponente ausgebildeten Gegenstück, vorzugsweise einem Vorsprung oder einer Ausnehmung, ist.

Gemäß einer Weiterbildung weist das fluidische Verbindungsstück wenigstens einen Kontaktabschnitt auf, mit dem ein Massekabel der Blutbehandlungsmaschine verbunden ist, das insbesondere mit dem elektrischen Bezugspotential verbunden ist.

Gemäß einer bevorzugten Weiterbildung ist der wenigstens eine Kontaktabschnitt als eine Kontaktfahne ausgebildet, auf die eine Flachsteckhülse des Massekabels aufgesteckt ist. So ist eine sichere und einfache elektrische Verbindung des Massekabels mit dem fluidischen Verbindungsstück realisiert.

Alternativ oder ergänzend ist ein Ringkabelschuh vorgesehen, welcher mit einer Schraube an dem Kontaktabschnitt befestigt ist, sodass die sichere und einfache elektrische Verbindung des Massekabels mit dem fluidischen Verbindungsstück realisiert ist.

Gemäß einer Weiterbildung haben die Fluidkanäle des fluidischen Verbindungsstücks Endabschnitte, an denen je einer der Abschnitte der jeweiligen Fluidleitung fluidisch angebunden ist.

Die Endabschnitte sind gemäß einer Weiterbildung zueinander diametral oder miteinander fluchtend angeordnet, sodass die mit dem Fluidkanal verbundene Fluidleitung ihre Richtung an dem fluidischen Verbindungsstück beibehält.

Alternativ können sich die Endabschnitte des jeweiligen Fluidkanals des fluidischen Verbindungsstücks zueinander angewinkelt erstrecken, sodass die mit dem jeweiligen Fluidkanal verbundene Fluidleitung ihre Richtung an dem fluidischen Verbindungsstück entsprechend dem Winkel ändert.

Um eine Montage der Fluidleitung, insbesondere ihres jeweiligen Abschnitts, an den Endabschnitten zu erleichtern, ist wenigstens einer der Endabschnitte stirnseitig konisch und/ oder angefast und/ oder angeschrägt ausgebildet. So kann die Fluidleitung, insbesondere der jeweilige Abschnitt, einfacher auf den Endabschnitt des Fluidkanals aufgeschoben werden, was die fluidische Verbindung vereinfacht.

Gemäß einer Weiterbildung hat der jeweilige Endabschnitt des Fluidkanals einen planen oder flachen Ringstirnflächenabschnitt, der den Konus und/ oder die Fase und/ oder die Schräge stirnseitig plan oder flach begrenzt oder beschneidet. Dadurch weist der jeweilige Endabschnitt des Fluidkanals stirnseitig keine scharfe Spitze oder Kante auf, sodass beim Aufschieben des Abschnitts der Fluidleitung auf den jeweiligen Endabschnitt des Fluidkanals die Fluidleitung nicht verletzt werden kann.

In vorteilhafter Weise ist das fluidische Verbindungsstück im Bereich der Endabschnitte der Fluidkanäle, auf die die Abschnitte der Fluidleitungen aufgeschoben sind, verrundet. Durch diese Verrundung sind in diesem Bereich keine scharfen Kanten vorhanden. Insbesondere sind hierbei Übergänge zwischen dem Konus und/ oder der Fase und/ oder der Schräge und/ oder der Ringstirnfläche verrundet.

Um auf die Endabschnitte des Fluidkanals aufgeschobene Abschnitte der Fluidleitungen gegen ein Abziehen zu sichern, weist wenigstens einer der Endabschnitte gemäß einer Weiterbildung eine außenumfänglich umlaufende Riffelung auf.

Alternativ oder ergänzend kann eine den aufgeschobenen Abschnitt umgreifende Schlauchsicherung vorgesehen sein, insbesondere eine Schlauchschelle, vorzugsweise ein "Snapper" mit in Eingriff bringbaren und spannbaren Verzahnungsabschnitten.

Vorzugsweise ist der aufgeschobene Abschnitt der Fluidleitung zumindest in radialer Richtung elastisch und ist formschlüssig mit der Riffelung verbunden.

Gemäß einer Weiterbildung hat das Gehäuse wenigstens eine Durchgangsausnehmung, die von einer fluidischen Durchgangstülle durchgriffen ist, an die innenraumseitig, sowie außenseitig je ein Abschnitt einer der Fluidleitungen fluidisch angebunden ist.

Gemäß einer Weiterbildung weist diese Gehäusedurchführung keine Potentialausgleichsfunktion auf und kann daher vorrichtungstechnisch einfacher ausgestaltet sein als eine Gehäusedurchführung mit Potentialausgleichsfunktion.

Gemäß einer bevorzugten Weiterbildung ist die fluidische Durchgangstülle als ein elektrischer Isolator ausgebildet. Das bietet Freiheit in der Ausgestaltung des Gehäuses, das dann elektrisch leitfähig oder elektrisch isolierend ausgebildet sein kann.

Alternativ ist die fluidische Durchgangstülle als ein elektrischer Leiter ausgebildet. In diesem Falle ist entweder das Gehäuse als elektrischer Isolator ausgebildet oder das Gehäuse ist elektrisch leitend und zwischen der fluidischen Durchgangstülle und dem Gehäuse ist ein Isolator angeordnet.

Jegliche Offenbarung bezüglich der Ausgestaltung und Anordnung des fluidischen Verbindungsstücks gemäß der vorbeschriebenen, offenbarungsgemäßen extrakorporalen Blutbehandlungsmaschine gilt auch für das nachfolgend beschriebene, offenbarungsgemäße, elektrisch leitfähige, fluidische Verbindungsstück - und umgekehrt.

Gemäß der vorliegenden Offenbarung ist ein elektrisch leitfähiges, fluidisches Verbindungsstück für eine oder einer extrakorporalen Blutbehandlungsmaschine vorgesehen. Insbesondere ist seine Anordnung an einem Gehäuse der extrakorporalen Blutbehandlungsmaschine vorgesehen oder es ist zur zumindest abschnittsweisen Anordnung in einem von einem Gehäuse umfangenen Innenraum der extrakorporalen Blutbehandlungsmaschine vorgesehen. Gemäß der Offenbarung hat das Verbindungstück wenigstens zwei elektrisch leitfähige Fluidkanäle, die jeweils zur fluidischen Anbindung von Abschnitten von einer von wenigstens zwei Fluidleitungen der Blutbehandlungsmaschine vorgesehen sind, und die insbesondere in baulicher Einheit zusammenfasst sind.

Gemäß einer bevorzugten Weiterbildung ist das Verbindungsstück einstückig ausgebildet.

Eine einstückige Ausbildung des Verbindungsstücks kann beispielsweise durch Urformen des Verbindungsstücks erfolgen.

Die Einstückigkeit des fluidischen Verbindungsstücks hat den Vorteil, dass mehrere Fluidführungen und deren elektrische Verbindungmöglichkeit mit dem elektrischen Bezugspotential in genau einem Körper/ in genau einem Stück funktional integriert sind. So wird auf vorrichtungstechnisch einfachste Weise die gemeinsame Erdung mehrerer Fluidleitungen ermöglicht.

Durch die Einstückigkeit oder einstückige Fertigung können Fugen, Spalte oder Übergänge - wie sie bei einer herkömmlichen Fertigung mittels Fügen oder Verbinden mehrerer Teile oder Stücke zu dem Verbindungsstück entstehen würden - entfallen oder zumindest in ihrer Anzahl reduziert sein und/ oder in ihrer Form optimiert ausgestaltet sein. Generell bilden Fugen, Spalte oder Übergänge ein Risiko, dass in sie Flüssigkeit, Staub oder Schmutz eindringt oder daran anhaftet. Gegenüber dem herkömmlichen durch Fügen oder Verbinden gefertigten Verbindungsstück ist beim offenbarungsgemäß einstückig ausgebildeten Verbindungsstück dieses Risiko aufgrund einer potentiell geringeren Anzahl und einer optimierten Form von Fugen, Spalten oder Übergänge minimiert.

Der Begriff der Einstückigkeit des Verbindungsstücks bedeutet im Rahmen der Offenbarung insbesondere, dass das Verbindungsstück ohne ein Verbindungsmittel oder Fügemittel gefertigt ist. Das heißt, es besteht insbesondere nicht aus Teilen oder Stücken, die miteinander formschlüssig oder kraftschlüssig oder stoffschlüssig verbunden sind, beispielsweise geschraubt oder geklebt oder geschweißt oder genietet oder anderweitig verbunden sind. In anderen Worten ist unter der Einstückigkeit des Verbindungsstücks insbesondere eine einmaterialige, integrale, monolithische Fertigung und Ausbildung des Verbindungsstücks zu verstehen.

Der Begriff der Einstückigkeit des Verbindungsstücks bedeutet im Rahmen der Offenbarung vorzugsweise, dass das Verbindungsstück homogen aus einem Material oder Stoff besteht.

Vorzugsweise ist das Verbindungsstück stoffeinstückig gefertigt, beispielsweise als Guss-, Spritzguss- oder additiv gefertigtes Bauteil.

Bevorzugt erfolgt die Fertigung mittels einem Metal Injection Moulding Verfahren (MIM).

Gemäß einer Weiterbildung sind Endabschnitte der wenigstens zwei Fluidkanäle stirnseitig konisch oder angefast oder angeschrägt ausgebildet, um ein Ansetzen und Aufziehen der Abschnitte der Fluidleitungen zu vereinfachen.

Ergänzend oder alternativ weist wenigstens einer der Endabschnitte der wenigstens zwei Fluidkanäle eine außenumfänglich umlaufende Riffelung auf. Über diese Riffelung ist der jeweilige Abschnitt der Fluidleitung gegen ein axiales Abziehen von dem Endschnitt, an dem er angebunden ist, gesichert oder das axiale Abziehen ist zumindest erschwert.

Gemäß einer Weiterbildung hat das Verbindungsstück einen Befestigungsabschnitt, der zur Befestigung des Verbindungsstücks an dem Gehäuse oder an einer im Innenraum des Gehäuses angeordneten, insbesondere mit dem elektrischen Bezugspotential verbundenen, Komponente der Blutbehandlungsmaschine vorgesehen und ausgestaltet ist.

Vorzugsweise sind die Fluidkanäle beidseitig des Befestigungsabschnitts angeordnet oder der Befestigungsabschnitt ist zentral oder mittig zwischen den Fluidkanälen vorgesehen. Die Halte- oder Lagerkräfte von angebundenen Fluidleitungen können dadurch ausgewogen, insbesondere symmetrisch, ins Verbindungsstück eingeleitet werden. Insbesondere kann die mechanische Belastung des Verbindungsstücks verringert und eine Befestigungskraft klein gehalten werden.

Alternativ dazu erstreckt sich der Befestigungsabschnitt seitlich von den wenigstens zwei Fluidkanälen, insbesondere in Form einer seitlich abstehenden Lasche. Mit seitlicher Erstreckung ist gemeint, dass der Befestigungsabschnitt an einer Seite des Verbindungstücks und die wenigstens zwei Fluidkanäle an der anderen Seite des Verbindungsstücks angeordnet sind.

Gemäß einer Weiterbildung erstreckt sich der Befestigungsabschnitt in einer Befestigungsrichtung über die wenigstens zwei elektrisch leitfähigen Fluidkanäle hinaus. Mit Befestigungsrichtung ist eine Richtung quer zu den Fluidkanälen und hin zu einer für die Befestigung des Verbindungsstücks vorgesehenen Befestigungsebene oder Befestigungsoberfläche gemeint.

Vorzugsweise erstreckt sich der Befestigungsabschnitt in der Befestigungsrichtung mit einem Überstand über die wenigstens zwei elektrisch leitfähigen Fluidkanäle hinaus.

Vorzugsweise erstreckt sich der Befestigungsabschnitt in der Befestigungsrichtung mit dem Überstand über einen Außenumfang der wenigstens zwei elektrisch leitfähigen Fluidkanäle hinaus.

Vorzugsweise ist der Überstand so bemessen, dass er größer ist als eine Wandstärke der mit den Fluidkanälen zu verbindenden Fluidleitungen.

Bevorzugt ist vom Befestigungsabschnitt ein sich in der Befestigungsrichtung erstreckender Sockel gebildet, über den die Fluidkanäle bei befestigtem Verbindungsstück beabstandet zur Befestigungsebene oder Befestigungsoberfläche angeordnet sind.

Gemäß einer Weiterbildung hat das Verbindungsstück wenigstens einen, vorzugsweise einen als eine Kontaktfahne ausgebildeten, Kontaktabschnitt, der zur Verbindung mit einem Massekabel, vorzugsweise zum Aufstecken einer Flachsteckhülse oder zur Befestigung eines Ringkabelschuhs des Massekabels, vorgesehen und ausgestaltet ist.

Ergänzend kann wenigstens ein weiterer Kontaktabschnitt vorgesehen sein, sodass wenigstens eine weitere Option zur Anbindung des Massekabels bereitgestellt ist. Dadurch kann die Bauraumlage des Verbindungsstücks optimal auf den Verlauf der Fluidleitungen abgestimmt und freier gewählt werden. Nachdem die Bauraumlage feststeht, kann dann das Massekabel an den hinsichtlich seiner Lage geeigneten Kontaktabschnitt des Verbindungsstücks angebunden werden.

### Kurzbeschreibung der Figuren

Die Offenbarung wird nachfolgend anhand bevorzugter Ausführungsformen mit Hilfe von Figuren näher erläutert. Es zeigen:
- Fig. 1: eine extrakorporale Blutbehandlungsmaschine gemäß einer ersten offenbarungsgemäßen Ausführungsform, in einer Ansicht von vorne;
- Fig. 2: einen Innenraum der extrakorporalen Blutbehandlungsmaschine gemäß Figur 1 mit zwei über ein fluidisches Verbindungsstück geerdeten Fluidleitungen und mit Gehäusedurchführungen der Fluidleitungen;
- Fig. 3: das fluidische Verbindungsstück, die geerdeten Fluidleitungen und die Gehäusedurchführungen gemäß Figur 2, in teilperspektivischer Darstellung;
- Fig. 4: ein fluidisches Verbindungsstück für die extrakorporale Blutbehandlungsmaschine gemäß Figur 1, gemäß einer zweiten offenbarungsgemäßen Ausführungsform, in einer perspektivischen Ansicht von oben;
- Fig. 5: das fluidische Verbindungsstück gemäß Figur 4, in einer perspektivischen Ansicht von unten; und
- Fig. 6: ein fluidisches Verbindungsstück für die extrakorporale Blutbehandlungsmaschine gemäß Figur 1, gemäß einer dritten offenbarungsgemäßen Ausführungsform, in einer perspektivischen Ansicht von oben.

Die Figuren sind schematischer Natur und sollen nur dem Verständnis der Offenbarung dienen. Gleiche Elemente sind mit denselben Bezugszeichen versehen. Die Merkmale der verschiedenen Ausführungsformen können untereinander ausgetauscht werden.

### Detaillierte Beschreibung bevorzugter Ausführungsformen

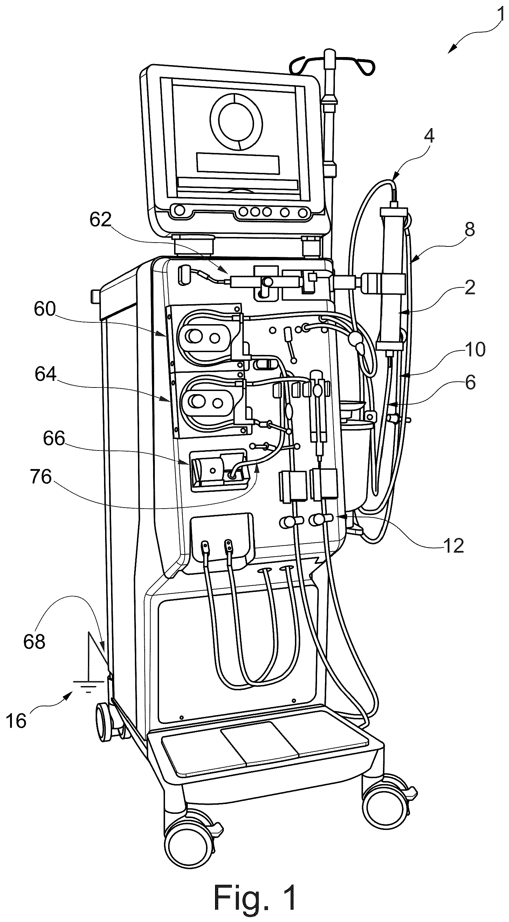

Figur 1 zeigt in einer schematischen Ansicht eine extrakorporale Blutbehandlungsmaschine 1 in Ausgestaltung als Hämodialysemaschine für eine extrakorporale Blutbehandlung von Blut eines Patienten gemäß einer bevorzugten Ausführungsform der vorliegenden Offenbarung.

Die extrakorporale Blutbehandlungsmaschine 1 hat einen Dialysator 2 mit einem Dialysierflüssigkeitskreislauf 8, 10 und einem extrakorporalen Blutkreislauf 4, 6. Der Dialysator 2 ist mittels Hohlfasern einer semipermeablen Membran in eine Dialysierflüssigkeitsseite und eine Blutseite separiert, wobei über die semipermeable Membran während der Blutbehandlung der notwendige Stoffaustausch erfolgt.

An einer Frontseite der extrakorporale Blutbehandlungsmaschine 1 sind eine Blutpumpe 60 und eine Heparinpumpe 62 vorgesehen. Die peristaltisch arbeitende Blutpumpe 60 ist mit einem Schlauchabschnitt des arteriellen Abschnitts 4 des extrakorporalen Blutkreislaufs 4, 6 gekoppelt. Die Heparinpumpe 62 ist fluidisch mit dem arteriellen Abschnitt 4 verbunden, um Heparin als Antikoagulanz zu dosieren.

Ergänzend zur Hämodialyse ist die extrakorporale Blutbehandlungsmaschine 1 für eine Hämofiltration und/ oder für eine Hämodiafiltration vorgesehen und ausgestaltet. Das dabei entzogene Wasser sollte dem Patienten als entsprechendes Volumen eines Substituats zugeführt werden. Zu diesem Zweck weist die extrakorporale Blutbehandlungsmaschine 1 eine Substitutionspumpe 64, sowie einen Substituatport 66 mit einem Substitutionsanschluss und einer Substituatleitung 76 auf.

Über eine Zulauf-Fluidleitung 8 ist ein Dialysierflüssigkeitseingang des Dialysators 2 mit einer innerhalb eines Gehäuse 12 angeordneten Dialysierflüssigkeitsbereitstellung fluidisch verbunden und über eine Rücklauf-Fluidleitung 10 ist ein Dialysatausgang des Dialysators 2 mit einer innerhalb des Gehäuse 12 angeordneten Dialysatsenke fluidisch verbunden. Über den extrakorporalen Blutkreislauf 4, 6 ist in einer bestimmungsgemäßen Blutbehandlungstherapie ein Patient über seinen Shunt arteriell und venös konnektiert (nicht dargestellt).

Grundlegend sind im Bereich der extrakorporalen Blutbehandlung derart flüssigkeitsführende Fluidleitungen, wie beispielsweise die dargestellte Zulauf-Fluidleitung 8 und Rücklauf-Fluidleitung 10 oder die Substituatleitung 76, erdungspflichtig und üblicherweise potentialausgeglichen, d.h. geerdet. In einem elektrischen Fehlerfall innerhalb der Blutbehandlungs- oder Dialysemaschine 1 wird so ein Patientenableitstrom eingehalten und das Patientenrisiko minimiert.

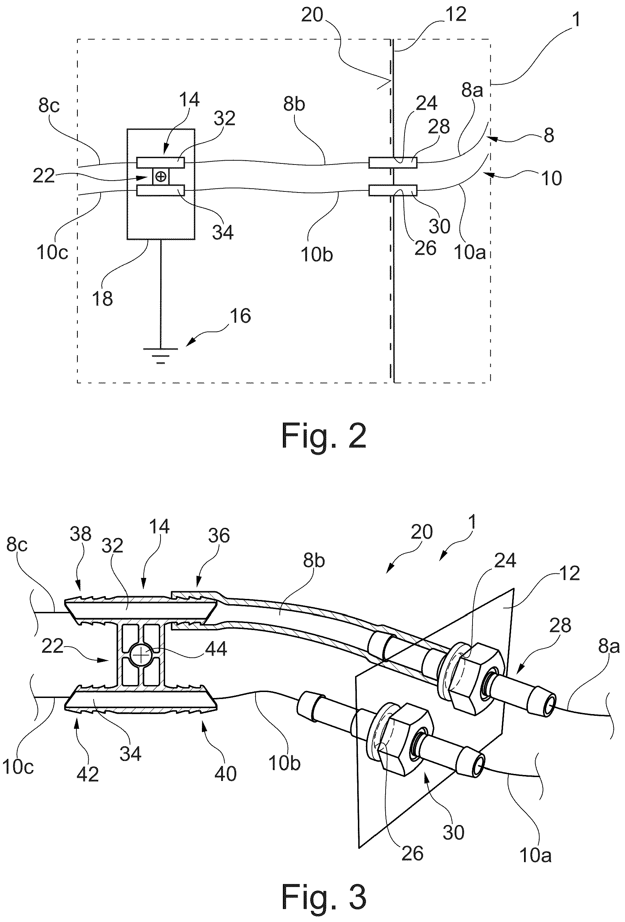

Für diesen Potentialausgleich hat die offenbarungsgemäße Blutbehandlungsmaschine 1 ein innerhalb des Gehäuses angeordnetes, elektrisch leitfähiges, fluidisches Verbindungsstück, das anhand der Figuren 2 bis 6 näher erläutert wird. An einer Rückseite der extrakorporalen Blutbehandlungsmaschine 1 ist für den Potentialausgleich ein Potenzialausgleichsanschluss 68 vorgesehen, der über ein Massekabel mit Masse 16 verbunden werden kann oder verbunden ist.

Figur 2 zeigt ein schematisches Detail, bzw. einen Teilschnitt, der Blutbehandlungsmaschine 1 gemäß Figur 1 mit einem vom Gehäuse 12 umfangenen (Gehäuse-) Innenraum 20. Von außerhalb, das heißt in Figur 2 von rechts, durchsetzen die Zulauf-Fluidleitung 8 und die Rücklauf-Fluidleitung 10 eine jeweilige Durchgangsausnehmung 24, 26 des Gehäuses 12, hinein in den Innenraum 20. In den Durchgangsausnehmungen 24, 26 ist jeweils eine als elektrischer Isolator ausgestaltete Durchgangstülle 28, 30 angeordnet, über die die jeweilige Fluidleitung 8, 10 gegen das Gehäuse 12 fluidisch gedichtet und elektrisch isoliert ist. Dabei ist ein erster Abschnitt 8a, 10a der jeweiligen Fluidleitung 8, 10 an einen außenseitigen Tüllenabschnitt und ein zweiter, innerer Abschnitt 8b, 10b der jeweiligen Fluidleitung 8, 10 an einen innenseitigen Tüllenabschnitt fluidisch angebunden.

Gemäß der Offenbarung ist in dem Innenraum 20 das elektrisch leitfähige, fluidische Verbindungsstück 14 über einen Befestigungsabschnitt 22 an einer mit Masse 16 verbundenen Komponente 18 der Blutbehandlungsmaschine 1 befestigt. Der Potentialausgleich bzw. die Erdung der beiden Fluidleitungen 8, 10 erfolgt somit über das Verbindungsstück 14, das weder an dem Gehäuse 12 angeordnet oder befestigt ist, noch in einer Ausnehmung des Gehäuses 12 angeordnet oder befestigt ist.

Zur fluidischen Anbindung der beiden Fluidleitungen 8, 10 hat das fluidische Verbindungsstück 14 in baulicher Einheit zwei Fluidkanäle 32, 34, die sich parallel, beidseitig des Befestigungsabschnitts 22 erstrecken. Gemäß Figur 2 sind an Endabschnitten des einen Fluidkanals 32 der zweite Abschnitt 8b und ein dritter Abschnitt 8c der Zulauf-Fluidleitung 8 fluidisch angebunden und an Endabschnitten des anderen Fluidkanals 34 sind der zweite Abschnitt 10b und ein dritter Abschnitt 10c der Rücklauf-Fluidleitung 10 fluidisch angebunden.

Figur 3 zeigt das Detail gemäß Figur 2 in einer weniger schematischen Darstellung, um die konstruktive Ausgestaltung des fluidischen Verbindungsstücks 14 und der Durchgangstüllen 28, 30 zu verdeutlichen.

Das fluidische Verbindungsstück 14 gemäß Figur 3 ist aus einem leitfähigen Metall einstückig mittels Spritzguss gefertigt. Als Fertigungsverfahren bietet sich vorzugsweise ein Metal Injection Moulding Verfahren (MIM) an.

Alternativ kann das fluidische Verbindungsstück 14 gemäß Figur 3 aus leitfähigem Kunststoff einstückig mittels Spritzguss gefertigt sein.

Miteinander fluchtende Endabschnitte 36, 38 und 40, 42 der beiden Fluidkanäle 32 und 34 weisen außenumfänglich eine Riffelung auf, welche jeweils von den elastisch ausgebildeten zweiten Abschnitten 8b, 10b und dritten Abschnitten 8c, 10c der Fluidleitungen 8, 10 formschlüssig umgriffen ist, was in Figur 3 allein für den zweiten Abschnitt 8b der Zulauf-Fluidleitung 8 detailliert dargestellt ist. Auf diese Weise sind die Abschnitte 8b, 8c und 10b, 10c am jeweiligen Fluidkanal 32 und 34 gegen Abzug gesichert festgelegt.

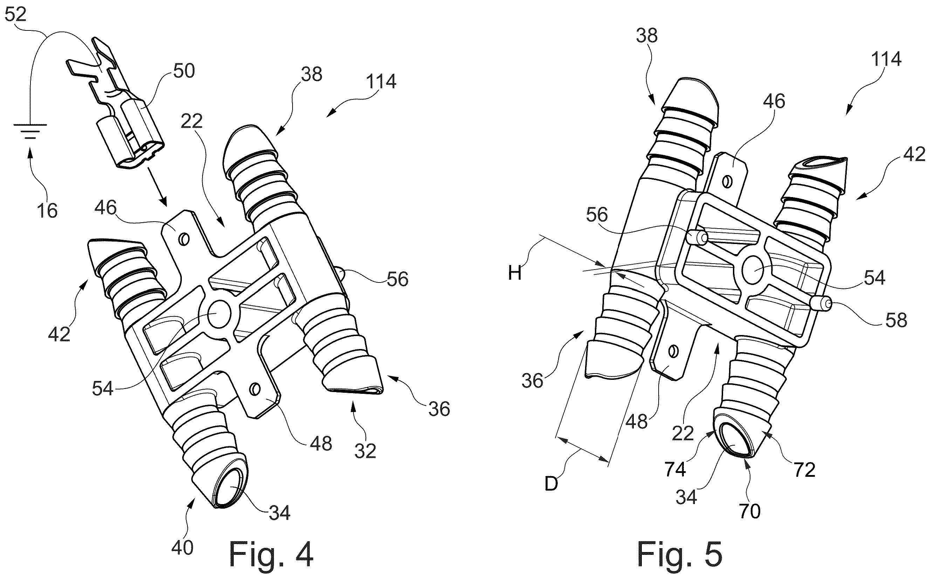

Die beiden Fluidkanäle 32, 34 sind symmetrisch, wobei Stirnseiten der Endabschnitte 36, 38, 40, 42 jeweils von lateral außen, hin zum Befestigungsabschnitt 22 abfallend angeschrägt sind, was die axiale Montage/ das axiale Aufschieben der Abschnitte 8b, 8c, 10b, 10c der beiden Fluidleitungen 8, 10 auf die Endabschnitte 36, 38, 40, 42 erleichtert. Im Bereich der Riffelung beträgt ein Außendurchmesser (vgl. D in Figur 5) der beiden Fluidkanäle 32, 34 jeweils 8,5 mm. Ein Innen- oder Nenn-Durchmesser der angebundenen Fluidleitungen 8, 10 ist kleiner als 8,5mm, sodass die aufgeschobenen, elastisch verformbaren Abschnitte 8b, 8c, 10b, 10c gegen versehentlichen Abzug gesichert auf der Riffelung sitzen.

Der Befestigungsabschnitt 22 hat eine als Durchgangsbohrung ausgebildete Durchgangsausnehmung, in der als Befestigungsmittel eine Schraube 44 eingesetzt ist, die mit einer Gewindebohrung der Komponente (vgl. 18, Fig. 2) verschraubt ist. Um das fluidische Verbindungsstück 14 an der Komponente 18 gegen Verdrehung zu sichern, ist rückwärtig zwischen dem Befestigungsabschnitt 22 und der Komponente eine Fächer- oder Zahnscheibe vorgesehen (verdeckt).

Die beiden Durchgangstüllen 28, 30 sind als baugleiche Standardtüllen aus Kunststoff elektrisch isolierend vorgesehen. In der jeweiligen Durchgangsausnehmung 24, 26 des Gehäuses 12 sind die Durchgangstüllen 28, 30 jeweils mittels einer gehäuseaußenseitig aufgeschraubten Überwurfmutter gegen ihren gehäuseinnenseitigen Kragen verspannt.

Die Figuren 4 und 5 zeigen ein zweites Ausführungsbeispiel eines fluidischen Verbindungsstücks 114 gemäß der Offenbarung. Es entspricht in weiten Teilen dem fluidischen Verbindungsstück 14 gemäß den Figuren 2 und 3, weshalb hier zunächst auf die Unterschiede zum ersten Ausführungsbeispiel eingegangen werden soll. Das fluidische Verbindungsstück 114 gemäß den Figuren 4 und 5 unterscheidet sich dabei vom ersten Ausführungsbeispiel in den Möglichkeiten der elektrischen Kontaktierung und der Verdrehsicherung.

Angenommen sei, dass das fluidische Verbindungsstück 114 - anstelle des vorbeschriebenen fluidischen Verbindungsstücks 14 - an der Komponente 18 gemäß Figur 2 befestigt sei. Entsprechend zeigt Figur 4 das fluidische Verbindungsstück 114 in einer perspektivischen Draufsicht. Ergänzend, bzw. abweichend vom vorbeschriebenen fluidischen Verbindungsstück 14 hat das fluidische Verbindungsstück 114 zwei als Kontaktfahnen 46, 48 ausgestaltete Kontaktabschnitte, die über eine aufsteckbare Flachsteckhülse 50 die Anbindung eines Potentialausgleichs- oder Masse-Kabels 52 ermöglichen. Die beiden Kontaktabschnitte/ Kontaktfahnen 46, 48 ermöglichen einen sehr flexiblen Anschluss des Massekabels 52.

Zum Zweck der Verdrehsicherung hat das fluidische Verbindungsstück 114 rückwärtig, das heißt, im Sinne der Figur 2 der Komponente 18 zugewandt, zwei zur zentralen Durchgangsausnehmung 54 des Befestigungsabschnitts 22 radial beabstandete Zapfen 56, 58 als Positioniermittel, von denen eine in eine entsprechende Ausnehmung der Komponente eintaucht (nicht dargestellt).

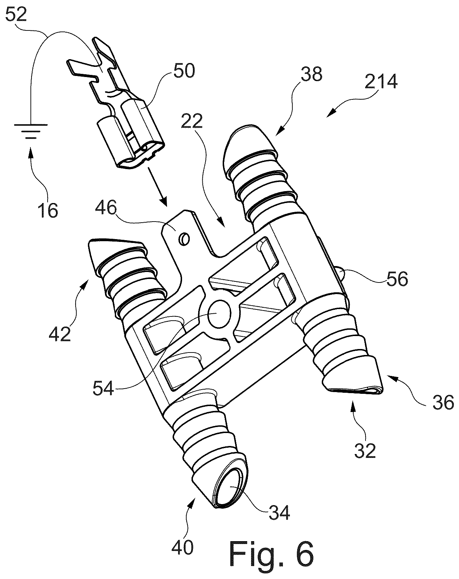

Figur 6 zeigt ein fluidisches Verbindungsstück 214 für die extrakorporale Blutbehandlungsmaschine 1 gemäß Figur 1 gemäß einer dritten offenbarungsgemäßen Ausführungsform, in einer perspektivischen Ansicht von oben. Abweichend vom Ausführungsbeispiel gemäß Figur 4 und 5 ist nur die eine Kontaktfahne 46 vorgesehen. Die Ausgestaltung des Verbindungsstücks 214 ist damit etwas einfacher als diejenige der Ausführungsform gemäß Figur 4 und 5 und der Anschluss des Massekabels 52 ist auf die eine Kontaktfahne 46 beschränkt.

Die drei Ausführungsbeispiele des Verbindungstücks 14; 114; 214 haben gemein, dass der Befestigungsabschnitt 22 in Befestigungsrichtung hin zu einer Befestigungsebene/ - fläche - also beispielsweise in Richtung hin zu einer Oberfläche der Komponente 18, an der das Verbindungstück 14; 114; 214 befestigt/ zu befestigen ist - einen Sockel ausbildet. Der Sockel weist einen Überstand (vgl. H in Figur 5) über den Außenumfang der Endabschnitte 36, 38, 40, 42 der Fluidkanäle 32, 34 auf. Der Überstand ist dabei so bemessen, dass er größer ist als eine Wandstärke der anzubindenden Fluidleitungen 8, 10. So ist bei befestigtem Verbindungsstück 14; 114; 214 das Aufschieben der Abschnitte 8b, 8c, 10b, 10c der Fluidleitungen 8, 10 auf die geriffelten Endabschnitte 36, 38, 40, 42 - und auch das Abziehen - auf einfache Weise möglich.

Für den Fall, dass die Abschnitte 8b, 8c, 10b, 10c der Fluidleitungen 8, 10 bereits auf die geriffelten Endabschnitte 36, 38, 40, 42 aufgeschoben sind, kann die Befestigung des Verbindungsstücks 14; 114; 214 auf einfache Weise erfolgen, weil die Wandung der aufgeschobenen Abschnitte 8b, 8c, 10b, 10c beim Befestigen aufgrund des ausreichend bemessenen Überstandes H nicht gequetscht werden muss.

Anhand der Figur 5 wird eine weitere Gemeinsamkeit der Ausführungsbeispiele des Verbindungstücks 14; 114; 214 beschrieben. Jeder Endabschnitt 36, 38, 40, 42 der Fluidkanäle 32, 34 hat endseitig einen Konus 72, der in Richtung nach lateral innen (hin zu einer Mittelebene des Verbindungstücks 14; 114; 214 von einer Schräge 74 abgeschnitten ist. Stirnseitig ist der Konus 72 von einem gegen die Schräge 74 angestellten, planen oder flachen Ringstirnflächenabschnitt 70 beschnitten.

Durch diesen Ringstirnflächenabschnitt 70 weist der Konus 72 stirnseitig (trotz der Schräge 74) keine scharfe Spitze oder Kante mehr auf. So ist sichergestellt, dass die Abschnitte 8b, 8c, 10b, 10c der Fluidleitungen 8, 10 beim Aufschieben auf den jeweiligen Endabschnitt 36, 38, 40, 42 der Fluidkanäle 32, 34 nicht verletzt werden.

### Bezugszeichenliste

- 1: Extrakorporale Blutbehandlungsmaschine
- 2: Dialysator
- 3: Dialysierflüssigkeitskreislauf
- 4, 6: extrakorporaler Blutkreislauf
- 8, 8a, 8b, 8c: Zulauf-Fluidleitung Dialysierflüssigkeit
- 10, 10a, 10b, 10c: Rücklauf-Fluidleitung Dialysierflüssigkeit
- 12: Gehäuse
- 14; 114; 214: fluidisches Verbindungsstück
- 16: Masse
- 18: Komponente
- 20: Innenraum
- 22: Befestigungsabschnitt
- 24, 26: Durchgangsausnehmung
- 28, 30: Durchgangstülle
- 32, 34: Fluidkanal
- 36, 38, 40, 42: Endabschnitt
- 44: Schraube
- 46, 48: Kontaktabschnitt
- 50: Flachsteckhülse
- 52: Potentialausgleichskabel
- 54: Durchgangsbohrung
- 56, 58: Positioniermittel
- 60: Blutpumpe
- 62: Heparinpumpe
- 64: Substitutionspumpe
- 66: Substituatport
- 68: Potenzialausgleichsanschluss
- 70: Ringstirnflächenabschnitt
- 72: Konus
- 74: Schräge
- 76: Substituatleitung

## Patentansprüche

1. Extrakorporale Blutbehandlungsmaschine (1), insbesondere Dialysemaschine, mit:
- Fluidleitungen (8, 10), insbesondere erdungspflichtigen, die jeweils in Abschnitte (8b, 8c, 10b, 10c) segmentiert vorgesehen und ausgestaltet sind, und
- elektrisch leitfähigen Fluidkanälen (32, 34), über die jeweils zwei Abschnitte (8b, 8c, 10b, 10c) der jeweiligen Fluidleitung (8, 10) fluidisch verbunden oder zumindest fluidisch verbindbar sind, wobei die Fluidkanäle (32, 34) zur elektrischen Verbindung mit einem elektrischen Bezugspotential (16) vorgesehen sind oder mit einem elektrischen Bezugspotential (16) elektrisch verbunden sind, **dadurch gekennzeichnet, dass** wenigstens zwei der Fluidkanäle (32, 34) zu einem elektrisch leitfähigen, fluidischen Verbindungsstück (14; 114; 214), insbesondere in baulicher Einheit, zusammengefasst sind.

2. Extrakorporale Blutbehandlungsmaschine (1) nach Anspruch 1, **dadurch gekennzeichnet, dass** das Verbindungsstück (14; 114; 214) einstückig ist oder einstückig gefertigt ist.

3. Extrakorporale Blutbehandlungsmaschine (1) nach Anspruch 1 oder 2, **dadurch gekennzeichnet, dass** das fluidische Verbindungsstück (14; 114; 214) zumindest abschnittsweise, vorzugsweise vollständig, in einem von einem Gehäuse (12) der Blutbehandlungsmaschine (1) umfangenen Innenraum (20) angeordnet ist.

4. Extrakorporale Blutbehandlungsmaschine (1) nach einem der Ansprüche 1 bis 3, **dadurch gekennzeichnet, dass** das Verbindungsstück (14; 114; 214) einen Befestigungsabschnitt (22) hat, über den es an einem Gehäuse (12) der Blutbehandlungsmaschine (1) befestigt ist, oder über den es an einer zumindest abschnittsweise in einem von einem Gehäuse (12) der Blutbehandlungsmaschine (1) umfangenen Innenraum (20) angeordneten, insbesondere mit dem elektrischen Bezugspotential (16) verbundenen, Komponente (18) der Blutbehandlungsmaschine (1) befestigt ist.

5. Extrakorporale Blutbehandlungsmaschine (1) nach Anspruch 4, **dadurch gekennzeichnet, dass** der Befestigungsabschnitt (22) eine Durchgangsausnehmung (54) und wenigstens ein zur Durchgangsausnehmung (54) beabstandetes Positioniermittel (56, 58) hat.

6. Extrakorporale Blutbehandlungsmaschine (1) nach einem der vorhergehenden Ansprüche, **dadurch gekennzeichnet, dass** das Verbindungsstück (114; 214) wenigstens einen Kontaktabschnitt (46, 48) hat, mit dem ein Massekabel (52) verbunden oder verbindbar ist.

7. Extrakorporale Blutbehandlungsmaschine (1) nach Anspruch 6, **dadurch gekennzeichnet, dass** der wenigstens eine Kontaktabschnitt als eine Kontaktfahne (46, 48) ausgebildet ist, auf die eine Flachsteckhülse (50) des Massekabels (52) aufgesteckt ist.

8. Extrakorporale Blutbehandlungsmaschine (1) nach einem der vorhergehenden Ansprüche, **dadurch gekennzeichnet, dass** die Fluidkanäle (32, 34) Endabschnitte (36, 38, 40, 42) haben, an denen je einer der Abschnitte (8b, 8c, 10b, 10c) der jeweiligen Fluidleitung (8, 10) fluidisch angebunden oder zumindest fluidisch anbindbar ist, wobei wenigstens einer der Endabschnitte (36, 38, 40, 42) stirnseitig konisch und/ oder angefast und/ oder angeschrägt ist und/ oder eine außenumfänglich umlaufende Riffelung hat.

9. Extrakorporale Blutbehandlungsmaschine (1) nach einem der vorhergehenden Ansprüche, **dadurch gekennzeichnet, dass** das Gehäuse (12) wenigstens eine Durchgangsausnehmung (24, 26) hat, die von einer fluidischen Durchgangstülle (28, 30) durchgriffen ist, an die innenraumseitig, sowie außenseitig je ein Abschnitt (8b, 8a, 10b, 10a) einer der Fluidleitungen (8, 10) fluidisch angebunden ist.

10. Elektrisch leitfähiges, fluidisches Verbindungsstück (14; 114; 214) für eine oder einer extrakorporalen Blutbehandlungsmaschine (1), insbesondere zur Anordnung an einem Gehäuse der extrakorporalen Blutbehandlungsmaschine (1) oder zur zumindest abschnittsweisen Anordnung in einem von einem Gehäuse (12) umfangenen Innenraum (20) der extrakorporalen Blutbehandlungsmaschine (1), **dadurch gekennzeichnet, dass** das Verbindungstück (14; 114; 214) wenigstens zwei elektrisch leitfähige Fluidkanäle (32, 34) hat, und diese insbesondere in baulicher Einheit zusammenfasst, die jeweils zur fluidischen Anbindung von Abschnitten (8b, 8c, 10b, 10c) von einer von wenigstens zwei Fluidleitungen (8, 10) der Blutbehandlungsmaschine (1) vorgesehen sind.

11. Elektrisch leitfähiges, fluidisches Verbindungsstück (14; 114; 214) nach Anspruch 10, **dadurch gekennzeichnet, dass** es einstückig ist oder einstückig, insbesondere stoffeinstückig, gefertigt ist.

12. Elektrisch leitfähiges, fluidisches Verbindungsstück (14; 114; 214) nach Anspruch 10 oder 11, **dadurch gekennzeichnet, dass** Endabschnitte (36, 38, 40, 42) der wenigstens zwei Fluidkanäle (32, 34) stirnseitig konisch und/ oder angefast und/ oder angeschrägt sind und/ oder mit einer außenumfänglich umlaufenden Riffelung ausgestaltet sind.

13. Elektrisch leitfähiges, fluidisches Verbindungsstück (14; 114; 214) nach einem der Ansprüche 10 bis 12, **dadurch gekennzeichnet, dass** ein Befestigungsabschnitt (22) vorgesehen ist, der zur Befestigung des Verbindungsstücks (14; 114; 214) an dem Gehäuse (12) oder an einer zumindest abschnittsweise im Innenraum (20) angeordneten, insbesondere mit dem elektrischen Bezugspotential (16) verbundenen, Komponente (18) der Blutbehandlungsmaschine (1) vorgesehen und ausgestaltet ist.

14. Elektrisch leitfähiges, fluidisches Verbindungsstück (14; 114; 214) nach Anspruch 13, **dadurch gekennzeichnet, dass** sich der Befestigungsabschnitt in einer Befestigungsrichtung, insbesondere quer zur Erstreckungsrichtung der wenigstens zwei elektrisch leitfähigen Fluidkanäle (32, 34), über die wenigstens zwei elektrisch leitfähigen Fluidkanäle (32, 34) hinaus erstreckt,

15. Elektrisch leitfähiges, fluidisches Verbindungsstück (14; 114; 214) nach einem der Ansprüche 10 bis 14, **dadurch gekennzeichnet, dass** es einen, vorzugsweise als eine Kontaktfahne (46, 48; 46) ausgebildeten, Kontaktabschnitt hat, der zur Verbindung mit einem Massekabel (52), vorzugsweise zum Aufstecken einer Flachsteckhülse (50) oder zur Befestigung eines Ringkabelschuhs des Massekabels (52), vorgesehen und ausgestaltet ist.
